# EUROPEAN PATENT APPLICATION

(11) **EP 2 641 603 A1**
(43) Date of publication of application: **25.09.2013**
(21) Application number: 11842188.2
(22) Date of filing: 09.11.2011
(51) Int. Cl.: A61K 31/58, A61K 31/255, A61P 25/00, A61P 27/02, A61P 29/00, A61P 43/00

(54) **PHARMACEUTICAL FORMULATION HAVING NEUROPROTECTIVE ACTIVITY**

(30) Priority: 15.11.2010 JP 2010255422
(71) Applicant: Kyushu University, National University Corporation, Fukuoka-shi Fukuoka 812-8581 (JP)
(72) Inventor: ISHIBASHI Tatsurou, Fukuoka-shi Fukuoka 812-8581 (JP); HISATOMI Toshio, Fukuoka-shi Fukuoka 812-8581 (JP); NOUTOMI Syouji, Fukuoka-shi Fukuoka 812-8581 (JP); ENAIDA Hiroshi, Fukuoka-shi Fukuoka 812-8581 (JP); KAGIMOTO Tadahisa, Fukuoka-shi Fukuoka 811-0215 (JP)
(74) Representative: Instone, Alicia Claire
(86) International application number: PCT/JP2011/075802
(87) International publication number: WO 2012/066994

(57) **Abstract**

[Problem] The purpose of the present invention is to provide a neuroprotective agent which is capable of effectively protecting intraocular cells including optic nerves and is effective as a composition for ophthalmic membrane staining in cases where removal of an ophthalmic membrane is carried out.

[Solution] The purpose is achieved by a neuroprotective agent which contains, as active ingredients: Brilliant Blue G, a pharmaceutically acceptable salt thereof or a harmaceutically acceptable solvate thereof; and triamcinolone acetonide, a pharmaceutically acceptable salt thereof or a pharmaceutically acceptable solvate thereof. This neuroprotective agent has ophthalmic membrane staining effect due to BBG and exhibits extremely high neuroprotective activity due to synergetic effect of Brilliant Blue G and triamcinolone acetonide.

## Description

### Technical Field

The present invention relates to a neuroprotective agent having a neuroprotective effect. In more detail, the present invention relates to a neuroprotective agent containing Brilliant Blue G, and triamcinolone acetonide.

### Background Art

In the specification of U.S. Patent No. 7731941 (Patent Literature 1), there is a disclosure that Brilliant Blue G (BBG) is effective as an ophthalmic membrane staining when the ophthalmic membrane removal is performed.

In U.S. Application Publication No. 2010-74884 (Patent Literature 2), there is a disclosure that BBG is an antagonist of a P2X receptor and inhibits or blocks the pathological action of ATP. Further, in the same Patent Literature, there is a disclosure that BBG has a neuroprotective effect.

On the other hand, in U.S. Application Publication No. 2004-65137 (Patent Literature 3), there is a disclosure of a preparation for injection containing steroid triamcinolone that is designed for the injection into the eye for the treatment of diseases of the eye. In this publication, there is a disclosure that a formulation that contains steroid triamcinolone and is commercially available is effective for the treatment of the allergic process and inflammatory process that relate to the eye and are serious and chronic (for example, ophthalmic zoster, iritis, iridocyclitis, chorioretinitis, diffuse posterior uveitis and choroiditis, optic neuritis, sympathetic ophthalmia, and anterior segment inflammation), and is also effective for the treatment of diabetic macular edema.

In U.S. Application Publication No. 2005-245497 (Patent Literature 4), there is a disclosure that steroid triamcinolone has a reducing effect for ocular angiogenesis.

In U.S. Application Publication No. 2006-9498 (Patent Literature 5), there is a disclosure that steroid triamcinolone is effective for the treatment of central retinal vein occlusion (CRVO), branch retinal vein occlusion (BRVO), cystoid macular edema (CME), diabetic macular edema (DME), diabetic macular retinopathy, uveitis, capillary inflammation, and age-related macular degeneration (ARMD).

In U.S. Application Publication No. 2005-181017 (Patent Literature 6), there is a disclosure that steroid triamcinolone is effective for the treatment of central retinal vein occlusion (CRVO), branch retinal vein occlusion (BRVO), cystoid macular edema (CME), diabetic macular edema (DME), diabetic macular retinopathy, uveitis, capillary inflammation, and age-related macular degeneration (ARMD).

The entirety of the known Literatures described above is incorporated in the specification by reference.

### Citation List

### Patent Literature

Patent Literature 1: U.S. Patent No. 7731941
Patent Literature 2: U.S. Application Publication No. 2010-74884
Patent Literature 3: U.S. Application Publication No. 2004-65137
Patent Literature 4: U.S. Application Publication No. 2005-245497
Patent Literature 5: U.S. Application Publication No. 2006-9498
Patent Literature 6: U.S. Application Publication No. 2005-181017

### Summary of Invention

### Technical Problem

The neuroprotective agent having an effect higher than that of the neuroprotective agent disclosed in the above-mentioned known Literatures was desired. In particular, when a removal of ophthalmic membrane or a vitreous staining was performed, an agent that can protect the optic nerve and effectively prevent the tissue from inflammation was desired.

An object of the present invention is to provide a neuroprotective agent that can effectively protect nerve cells. An object of the present invention is particularly to provide an optic neuroprotective agent.

An object of the present invention is to provide a neuroprotective agent that can effectively protect intraocular cells including optic nerve, and further is effective as a composition for staining an ophthalmic membrane when the ophthalmic membrane removal is performed.

An object of the present invention is to provide a neuroprotective agent that can effectively protect intraocular cells including optic nerve, and further is effective as a staining composition for staining the vitreous body.

An object of the present invention is to provide a neuroprotective agent having high anti-inflammatory effect.

### Solution to Problem

The present invention is based on the findings obtained from Examples, that is, an agent containing Brilliant Blue G and triamcinolone acetonide exerts an extremely effective neuroprotective effect due to the synergistic effect.

The present invention relates to a neuroprotective agent. The neuroprotective agent contains as an active ingredient Brilliant Blue G, a pharmaceutically acceptable salt thereof, or a pharmaceutically acceptable solvate thereof. Further, the neuroprotective agent contains as an active ingredient triamcinolone acetonide, a pharmaceutically acceptable salt thereof, or a pharmaceutically acceptable solvate thereof.

The agent is used particularly as a protective agent of the optic nerve.

The agent is used also as a staining composition for staining an ophthalmic membrane when performing the ophthalmic membrane removal.

The agent is used also as a staining composition for staining vitreous.

The agent can be used also as an anti-inflammatory agent.

### Advantageous Effects of Invention

The present invention can provide a neuroprotective agent that can effectively protect nerve cells. The present invention can provide particularly an optic neuroprotective agent.

The present invention can provide a neuroprotective agent that can effectively protect intraocular cells including the optic nerve, and further is effective as a composition for staining an ophthalmic membrane when ophthalmic membrane is removed.

The present invention can provide a neuroprotective agent that can effectively protect intraocular cells including the optic nerve, and further is effective as a staining composition for staining the vitreous body.

The present invention can provide a neuroprotective agent having high anti-inflammatory effect.

### Brief Description of the Drawings

Fig. 1 is fluorescence pictures (pictures alternative to the drawing) for which a cell-staining dye was used.
Fig. 2 is a graph showing the cell viability.

### Description of Embodiment

The first aspect of the present invention relates to a neuroprotective agent. An example of the neuroprotective agent is a protective agent of optic nerve. The neuroprotective agent can protect the intended nerves by topically administering to the nerves to be targeted. Therefore, the neuroprotective agent of the present invention is effective for the prevention and treatment of diseases of the nervous system, and further can be used during surgical procedures. In particular, the neuroprotective agent of the present invention can protect the nerve cells from damage by blocking the nerve cells. The agent has a dye-affinity, therefore, the places where the agent was administered can be clearly distinguished from the places where the agent was not administered, and further the agent has a high anti-inflammatory effect. Therefore, forgetting the administration of the agent can be effectively prevented.

An example of the nerve cells that can be protected by the neuroprotective agent of the present invention is optic nerves. In addition, the nerve cells that can be protected by the neuroprotective agent of the present invention may be neurons in nerves of the central nervous system (CNS) (for example, the cerebral or spinal cord neurons, or the retinal ganglion cells (RGC)), and in nerves of the peripheral nervous system (PNS) (for example, the autonomic nerves, the spinal nerves, or the cerebral nerves). Examples of nerve damages include hydrocephalus, edema, physical and compression injuries, excitotoxic injury, and ischemia. The neuroprotective agent of the present invention can effectively prevent and treat these nerve damages.

The neuroprotective agent basically contains two active ingredients. The first active ingredient is Brilliant Blue G, a pharmaceutically acceptable salt thereof, or a pharmaceutically acceptable solvate thereof. Further, the second active ingredient is triamcinolone acetonide, a pharmaceutically acceptable salt thereof, or a pharmaceutically acceptable solvate thereof.

Brilliant Blue G (BBG) is commercially available, therefore, BBG can be utilized by purchasing the BBG. Furthermore, the pharmaceutically acceptable salt of BBG, and the pharmaceutically acceptable solvate of BBG can be obtained by a known method. In addition, hereinafter, these are collectively also referred to as simply BBG. For example, these can be produced by a method disclosed in the specification of U.S. Patent No. 6,057,160, and this literature is incorporated herein by reference. The dosage of BBG can be adjusted according to known literatures. Further, the BBG can be formulated according to a method disclosed in the known literatures described above.

Triamcinolone acetonide is commercially available, therefore, triamcinolone acetonide can be utilized by purchasing the triamcinolone acetonide. Furthermore, the pharmaceutically acceptable salt of triamcinolone acetonide, and the pharmaceutically acceptable solvate of triamcinolone acetonide can be obtained by a known method. In addition, hereinafter, these are collectively also referred to as simply triamcinolone acetonide. The dosage of triamcinolone acetonide can be adjusted according to known literatures. Further, the triamcinolone acetonide can be formulated, according to a method disclosed in the known literatures described above.

Examples of the pharmacologically acceptable salt include an inorganic base, ammonia, an organic base, inorganic acid, organic acid, basic organic acid, a salt composed of halogen ions and the like, and an internal salt. Examples of the inorganic base include an alkali metal (Na, K, and the like) and an alkaline earth metal (Ca, Mg, and the like). Examples of the organic base include trimethylamine, triethylamine, choline, procaine, and ethanolamine. Examples of the inorganic acid include hydrochloric acid, hydrobromic acid, sulfuric acid, nitric acid, and phosphoric acid. Examples of the organic acid include p-toluenesulfonic acid, methanesulfonic acid, formic acid, trifluoroacetic acid, and maleic acid. Examples of the basic amino acid include lysine, arginine, ornithine, and histidine.

An example of the pharmaceutically acceptable solvate is a hydrate. An example of the hydrate is a monohydrate.

The second aspect of the present invention is a neuroprotective agent having also a staining application for staining an ophthalmic membrane when ophthalmic membrane is removed. That is, the agent of this aspect has also an application as a staining composition for staining an ophthalmic membrane when the ophthalmic membrane removal is performed, while exerting the high function as a neuroprotective agent that was described above. That is, as disclosed in Patent Literature 1, BBG is effective as a staining composition for staining an ophthalmic membrane when the ophthalmic membrane removal is performed. Further, as in the present invention, by containing triamcinolone acetonide, a synergistic effect of BBG and triamcinolone acetonide is generated, as a result, an extremely high neuroprotective effect can be exerted. Therefore, when the agent of the present invention is used for staining an ophthalmic membrane when ophthalmic membrane is removed, an extremely high dye-affinity and an extremely high neuroprotective effect can be exerted. In other words, the present invention also provides a staining composition for staining an ophthalmic membrane when the ophthalmic membrane removal is performed, which has a neuroprotective effect.

According to one of preferred embodiments of the present invention, the above-described staining composition can be used as a surgical adjuvant in ophthalmic surgeries of the ocular disease, for example, vitreo-retinal disease such as macular hole, retinal detachment due to hymyopic macular hole, epiretinal membrane, proliferative diabetic retinopathy, diabetic macular edema, proliferative vitreoretinopathy, and specific cataracts such as hypermature cataract and congenital cataract; split-thickness corneal transplantation; and the like. According to the staining composition of the present invention, the difficult-to-see ophthalmic membranes can be more clearly confirmed, and the safety during surgical procedures can be improved.

In a further preferred embodiment of the present invention, the staining composition can be used for staining an ophthalmic membrane, and more preferably, for staining the internal limiting membrane and/or the anterior capsule.

Further, according to one of embodiments of the present invention, the staining composition of the present invention can be used in combination with a pharmacologically acceptable carrier. The composition can be prepared as a set of a dissolving solution and drug powders, or as a form of a solution filled up in a syringe, and as a gel-state solution in combination with hyaluronic acid. Most preferably, the composition is prepared as a solution, but it is not necessarily limited thereto.

According to one of embodiments of the present invention, the above-mentioned staining composition can be prepared as a pharmaceutically acceptable solution, but it is not necessarily limited thereto. This is due to the characteristics of BBG, that is, the BBG can be directly and easily dissolved in an intraocular cleansing solution, and can be sterilized with a syringe filter.

Further, according to one of embodiments of the present invention, the above-mentioned staining composition can be prepared as a solution in which the composition is dissolved in an intraocular irrigating solution, a balanced salt solution (BSS), a physiological saline solution, or most preferably, OPEGUARD-MA (Senjyu Pharmaceutical Co., Ltd., Osaka, Japan) that is an intraocular irrigating solution (intraocular cleansing solution). However, it is not necessarily limited this form.

Furthermore, according to one of embodiments of the present invention, it is preferable to have the osmotic pressure in the vicinity of 298 mOsm. According to this embodiment, the staining composition of the present invention has the osmotic pressure equal to that of physiological saline solution. It was previously reported that disorders in the retinal pigment epithelium due to Indocyanine green (ICG) may be possibly derived from the hypoosmolality of the solution. In relation to this, the staining composition in one of embodiments of the present invention has an excellent effect, in the point of not causing tissue disorders that relate to cell expansion or dehydration (cell omission, cell death, or the like), such as disorders caused by the differences in the osmotic pressure in the retinal pigment epithelium.

In one of preferred embodiments of the present invention, the staining composition of the present invention desirably has neutral pH, that is, a pH in the vicinity of pH = 7.4.

In relation to an agent of this aspect, a method in which the agent is administered to an eye of a patient to stain the ophthalmic membrane and remove the stained ophthalmic membrane is provided. This method includes a step of preparing a staining composition containing BBG and triamcinolone acetonide, a step of staining an ophthalmic membrane using the staining composition with a predetermined concentration, and a step of removing the stained ophthalmic membrane. That is, the agent of this aspect can be used as a surgical adjuvant for ophthalmic surgeries. As one of preferred embodiments of the present invention, the above-mentioned ophthalmic surgeries are performed for the eye of mammals, and more preferably, for the eye of humans.

Further, in one of embodiments of the present invention, the above-mentioned staining composition and/or a staining method that utilizes the staining composition, can be suitably used as part of ophthalmic surgeries. According to the preferred embodiments of the present invention, the ophthalmologic surgeries described previously are surgeries for the treatment of macular hole, retinal detachment due to myopic macular hole, epiretinal membrane, proliferative diabetic retinopathy, diabetic macular edema, proliferative vitreoretinopathy, specific cataracts such as hypermature cataract and congenital cataract, split-thickness corneal transplantation, and the like, and most preferably, for vitreo-retinal disease (in particular macular hole or epiretinal membranes (ERMS)), and cataract.

In relation to the agent of this aspect, use of the BBG and triamcinolone acetonide to produce a staining composition for the treatment of ophthalmic diseases can be provided. In particular, the present invention can also provide the use of the BBG and triamcinolone acetonide to produce a staining composition for the removal of ophthalmic membrane.

The third aspect of the present invention relates to a neuroprotective agent having also an application for staining vitreous. That is, the agent of this aspect, by containing BBG and triamcinolone acetonide, has also an application as a staining composition for staining vitreous while exerting the high function as a neuroprotective agent that was described above. The agent of this aspect is similar to that of the second aspect described above, therefore, the description is cited. That is, the present invention also provides a method in which the agent is administered into the vitreous body or on the surface of the vitreous body to stain the vitreous body of a patient. Further, the present invention also provides the use of the BBG and triamcinolone acetonide to produce a staining composition for staining vitreous. In other words, the present invention also provides a staining composition having a neuroprotective effect for staining vitreous.

The forth aspect of the present invention relates to a neuroprotective agent that functions as an anti-inflammatory agent. That is, the agent of the present invention has an anti-inflammatory function, and further has an extremely high neuroprotective effect by generating a synergistic effect of BBG and triamcinolone acetonide. That is, the agent of this aspect is effective for the prevention and treatment of ocular inflammation. The agent is effective for the prevention and treatment of conjunctivitis, keratitis, uveitis, scleritis, and retinitis.
Examples of the conjunctivitis include allergic conjunctivitis, keratoconjunctivitis sicca, acute viral conjunctivitis, epidemic keratitis, pharyngoconjunctival fever, acute hemorrhagic conjunctivitis, chlamydial conjunctivitis (trachoma), bacterial conjunctivitis, and gonococcal conjunctivitis.
Examples of the keratitis include corneal ulcer, bacterial corneal ulcer, catarrhal corneal ulcer, Mooren's ulcer, keratomycosis, herpes simplex keratitis, herpes zoster keratitis, Acanthamoeba keratitis, luetic interstitial keratitis, and superficial punctate keratitis.
Examples of the uveitis include Behcet's disease, sarcoidosis, Harada's disease, sympathetic ophthalmia, acute anterior uveitis (AAU), uveitis due to adult T-cell leukemia virus (HTLV-I associated uveitis (HAU)), retina conjunctivitis due to Toxocara canis, and Kirisawa type uveitis (acute retinal necrosis).
An example of the scleritis is episcleritis.
An example of the retinitis is retinal vasculitis.

The agent of this aspect is effective for the prevention and treatment of inflammation in a region other than the eye. Therefore, the agent is effective as an inflammation inhibitor during surgical procedures. Further, the agent is effective for the prevention and treatment of rheumatism and osteoarthritis. The agent of this embodiment has a dye-affinity, therefore, the places where the agent was administered can be clearly distinguished from the places where the agent was not administered, and further the agent has a high anti-inflammatory effect. Therefore, forgetting the administration of the agent can be effectively prevented. Further, the agent not only has simply a staining function, but also has an extremely high neuroprotective effect and cytoprotection due to the generation of a synergistic effect of BBG and triamcinolone acetonide.

Next, the dosage of BBG in the agent of the present invention is explained. The agent of the present invention desirably contains BBG at a concentration of 0.1 to 10 mg/ml, preferably 0.1 to 1.0 mg/ml, and most preferably 0.1 to 0.25 mg/ml. With the BBG at a low concentration of 0.25 mg/ml in a staining composition, sufficient staining and a neuroprotective effect can be obtained. In addition, also when the agent of the present invention is used as a neuroprotective agent, the agent may be administered locally to the place to be treated, and BBG may be administered in an amount of 1 µg or more to 10 mg or less at a time. Specifically, the BBG may be administered in an amount of 10 µg at a time.

Next, the dosage of triamcinolone acetonide in the agent of the present invention is explained. The agent of the present invention preferably contains triamcinolone acetonide in an amount of 1 mg/ml or more to 500 mg/ml or less. Further, triamcinolone acetonide may be administered in an amount of 1 µg or more to 0.5 g or less at a time. Specifically, the triamcinolone acetonide may be administered in an amount of 0.1 g at a time.

The dosage form of the agent of the present invention is not particularly limited. An example of the dosage form is an injection. Further, by using the dosage form disclosed in the Patent Literature described above, the agent can be administered locally into the eye. In order to produce the injection, a known carrier (water and the like) and active ingredients are mixed, and the mixture may be enclosed in a syringe. As the production method of dosage form, a known method can be appropriately used.

The neuroprotective agent of the present invention may be administered orally. In this case, the agent can be formulated by mixing a known carrier with the BBG and triamcinolone acetonide that are the active ingredients of the agent, and then tableting the mixture by a tableting machine.

Specifically, when the agent of the present invention is used as a stain, the agent may be administered to the place to be treated in an appropriate amount. For example, the agent of the present invention that is an injection may be administered in an amount of 1 cc or more to 1 ml or less. When the agent of the present invention is used as a neuroprotective agent or an anti-inflammatory agent, the agent may be administered for example, 1 to 3 times per day for a predetermined period.

### Example 1

### Neuroprotective effect test in primary culture of retinal neuron cells by simultaneous administration of Brilliant Blue G and triamcinolone acetonide

Mouse retina was collected, and was primary cultured in a Neurobasal A medium. After 10 µmol/l of Brilliant Blue G and 0.2 mg/ml of triamcinolone acetonide were administered separately or simultaneously, nutrient starvation stimulation was performed in a B27 supplement-free medium, living cells were fluorescently labeled with Calcein AM, and the cell viability was evaluated.

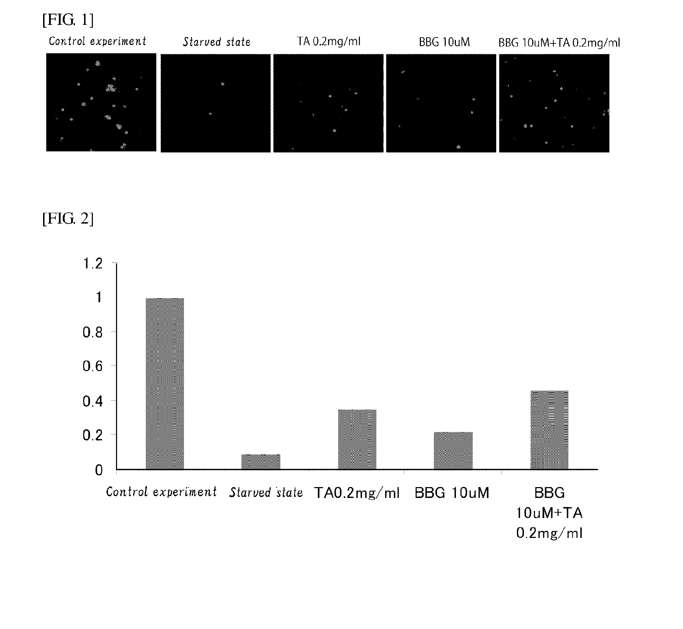

Fluorescence pictures for which a cell-staining dye was used are shown in Fig. 1. Further, the cell viabilities that were evaluated based on Fig. 1 are shown in Fig. 2. From Fig. 1, extremely high increase of the cell viability after the nutrient starvation stimulation was recognized in the mouse retina to which BBG and triamcinolone acetonide were administered simultaneously as compared with that in the mouse retina to which BBG and triamcinolone acetonide were administered separately. This means that by containing BBG and triamcinolone acetonide, a synergistic effect is generated, and thus the agent of the present invention has an extremely high neuroprotective effect.

### Industrial Applicability

The agent of the present invention can be utilized in the pharmaceutical industry.

## Claims

1. A neuroprotective agent comprising, as an active ingredient:
Brilliant Blue G, a pharmaceutically acceptable salt thereof, or a pharmaceutically acceptable solvate thereof; and
triamcinolone acetonide, a pharmaceutically acceptable salt thereof, or a pharmaceutically acceptable solvate thereof.

2. The neuroprotective agent according to claim 1, being a protective agent of an optic nerve.

3. The neuroprotective agent according to claim 1, further being used as a staining composition for staining an ophthalmic membrane when the ophthalmic membrane removal is performed.

4. The neuroprotective agent according to claim 1, further being used as a staining composition for staining vitreous.

5. The neuroprotective agent according to claim 1, further being used as an anti-inflammatory agent.
